# EUROPEAN PATENT APPLICATION

(11) **EP 4 799 683 A1**
(43) Date of publication of application: **02.09.2026**
(21) Application number: 26160298.1
(22) Date of filing: 24.02.2026
(51) Int. Cl.: A61N 5/10

(54) **RADIATION TREATMENT PLANNING MULTI-CRITERIA OPTIMIZATION METHOD AND APPARATUS**

(30) Priority: 26.02.2025 US 202519064001
(71) Applicant: Siemens Healthineers International AG, 6312 Steinhausen (CH)
(72) Inventor: KUUSELA, Esa, 00100 Helsinki (FI); SUHONEN, Pauli, 00100 Helsinki (FI); KAUPPINEN, Juha, 00100 Helsinki (FI)
(74) Representative: Mathisen & Macara LLP

(57) **Abstract**

A control circuit (101) accesses (201) an optimized radiation treatment plan that was developed using a corresponding utility function (302) that corresponds to a plurality of metrics and generates an approximation of the corresponding utility function. The control circuit (101) can then repeatedly selectively perturb (206) the approximation, each time with respect to only a different subset of the plurality of metrics, to generate a corresponding plurality of perturbed utility functions, and generate (207) a plurality of supplemental optimized radiation treatment plans, each as a function of a corresponding different one of the plurality of perturbed utility functions. The control circuit (101) can then employ (208) the plurality of supplemental optimized radiation treatment plans along with the optimized radiation treatment plan as a multi-criteria optimization workspace for a user to explore when selecting a particular optimized radiation treatment plan (113) to employ when administering therapeutic radiation to a particular patient (104).

## Description

### TECHNICAL FIELD

These teachings relate generally to an apparatus for treating a patient's planning target volume with energy pursuant to an energy-based treatment plan and more particularly to a method and/or apparatus for optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Multi-criteria optimization refers to a technique that aims to simultaneously balance and optimize multiple conflicting treatment objectives to achieve an optimal therapeutic outcome. This can involve the adjustment of various treatment parameters, such as radiation dose distribution, to maximize tumor control while minimizing irradiation of surrounding healthy tissues and organs. Multi-criteria optimization typically provides a set of Pareto optimal solutions in what is sometimes called the multi-criteria optimization workspace. These solutions represent good possible compromises and the workspace allows clinicians to select a most suitable treatment plan tailored to the specific needs and conditions of the patient.

### SUMMARY OF INVENTION

In accordance with a first aspect of the invention, there is provided a method as defined by claim 1.

In accordance with a second aspect of the invention, there is provided an apparatus as defined by claim 8.

In accordance with a third aspect of the invention, there is provided a non-transitory computer-readable medium as defined by claim 15.

Optional features are defined by the dependent claims.

### BRIEF DESCRIPTION OF DRAWINGS

Various needs are at least partially met through provision of the radiation treatment planning multi-criteria optimization method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a graph as configured in accordance with various embodiments of these teachings;
FIG. 4 comprises a graph as configured in accordance with various embodiments of these teachings; and
FIG. 5 comprises a graph as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments provide for a control circuit accessing an optimized radiation treatment plan that was developed using a corresponding utility function that corresponds to a plurality of metrics and generating an approximation of the corresponding utility function. The control circuit can then repeatedly selectively perturb the approximation, each time with respect to only a different subset of the plurality of metrics, to generate a corresponding plurality of perturbed utility functions, and generate a plurality of supplemental optimized radiation treatment plans, each as a function of a corresponding different one of the plurality of perturbed utility functions. The control circuit can then employ the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient.

By one approach, the aforementioned plurality of metrics can include at least one of a metric that corresponds to a prescribed clinical goal and a metric that corresponds to a preference of a treatment facility that may administer the therapeutic radiation to the particular patient.

By one approach, the aforementioned generation of the plurality of supplemental optimized radiation treatment plans can comprise generating the plurality of supplemental optimized radiation treatment plans without relying on a quadratic cost function.

By one approach, the aforementioned different subset of the plurality of metrics can comprise only a single different one of the plurality of metrics. If desired, all of the plurality of metrics can be individually used to create the corresponding plurality of perturbed utility functions.

By one approach, the aforementioned generation of the approximation of the corresponding utility function can comprise determining a tangent line at a particular point on a contour that corresponds to the optimized radiation treatment plan that was developed using the corresponding utility function that corresponds to the plurality of metrics. If desired, perturbing the approximation can comprise angularly perturbing the aforementioned tangent line with respect to the particular point. By one approach, these teachings will accommodate accessing a perturbation constraint, in which case the aforementioned perturbing of the approximation by angularly perturbing the tangent line with respect to the particular point can include limiting angular perturbation as a function of the perturbation constraint.

By one approach, the aforementioned generation of the approximation of the corresponding utility function can comprise generation of a linear approximation of the corresponding utility function. By another approach, the aforementioned generation of the approximation of the corresponding utility function can comprise generation of a non-linear approximation of the corresponding utility function.

If desired, these teachings will further accommodate presenting, via a user interface, an opportunity to select that perturbation constraint and then receiving input, via that user interface, which comprises a selection of the perturbation constraint to provide a user-selected perturbation constraint, in which case the aforementioned wherein accessing of the perturbation constraint can comprise accessing the user-selected perturbation constraint.

By one approach, these teachings will optionally accommodate administering therapeutic radiation to the particular patient as a function of a selected one of the optimized radiation treatment plans from the multi-criteria optimization workspace.

By one approach, these teachings can comprise a non-transitory computer-readable medium having a computer program that itself comprises instructions that, when the computer program is executed by a computer/processor, causes the computer/processor to carry out any of the steps, function, and/or activities described herein, including accessing an optimized radiation treatment plan that was developed using a corresponding utility function that corresponds to a plurality of metrics and generating an approximation of the corresponding utility function, repeatedly selectively perturbing the approximation, each time with respect to only a different subset of the plurality of metrics, to generate a corresponding plurality of perturbed utility functions, and generating a plurality of supplemental optimized radiation treatment plans, each as a function of a corresponding different one of the plurality of perturbed utility functions. The resultant plurality of supplemental optimized radiation treatment plans, along with the optimized radiation treatment plan, can then be used in a multi-criteria optimization workspace for a user to explore when selecting a particular optimized radiation treatment plan to employ when administering therapeutic radiation to a particular patient.

So configured, these teachings can provide for calculating a set of radiation treatment plans that span a part of, e.g. a relevant part of, the Pareto surface. This may be for the case when the utility function is a function of, e.g. an arbitrary (albeit well-behaving continuous) function of, the vector of corresponding metrics that are the same metrics that are going to be used in the multi-criteria optimization workflow. It will be further appreciated that these teachings can serve in these regards without the need to maintain a quadratic cost function during the optimization process, thus simplifying and reducing computational loading and reducing corresponding time requirements.

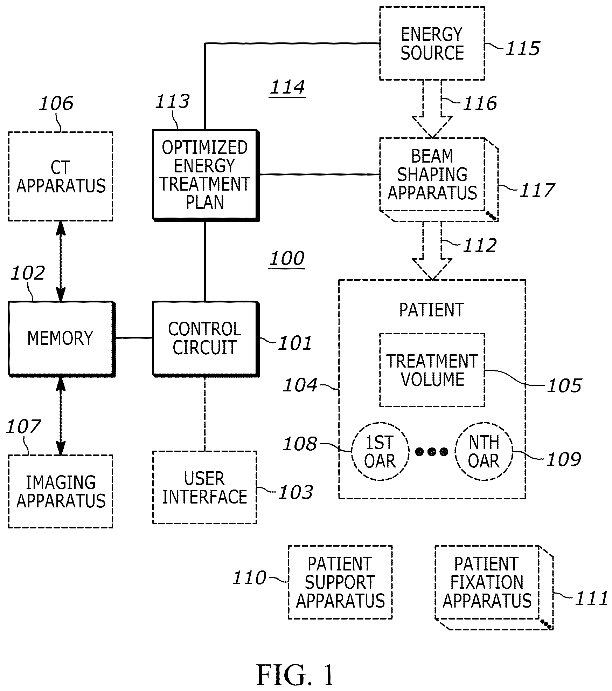

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM)))).

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

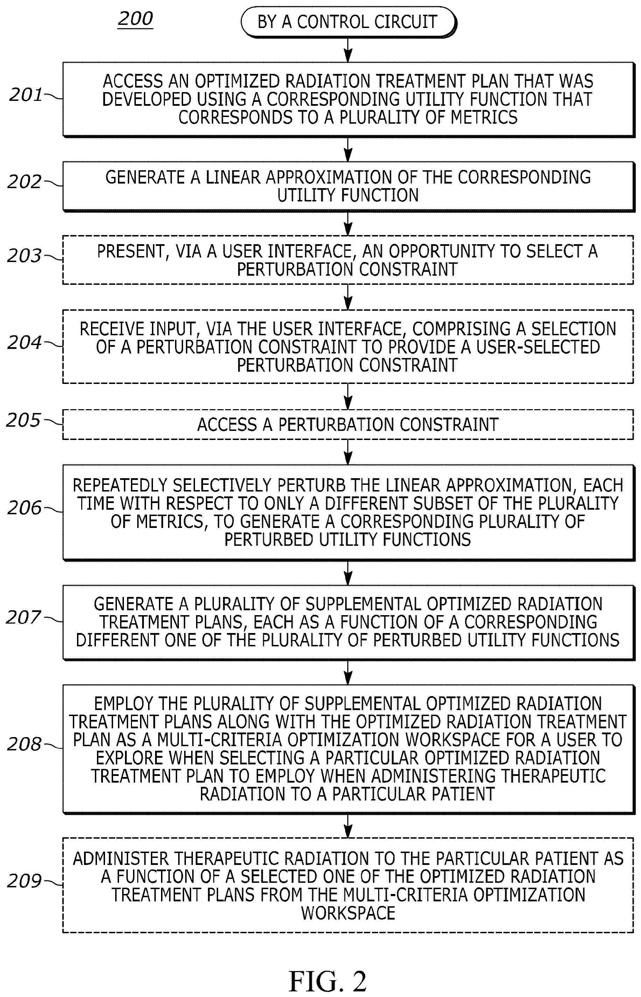

Referring now to FIG. 2, a process 200 that can be carried out, for example, in conjunction with the above-described application setting (and more particularly via the aforementioned control circuit 101) will be described. Generally speaking, this process 200 serves to facilitate generating a plurality of supplemental optimized radiation treatment plans that can be used in a multi-criteria optimization workspace to thereby facilitate selecting a particular radiation treatment plan with which to treat a particular patient with therapeutic radiation using a particular radiation treatment platform per that selected optimized radiation treatment plan.

At block 201, this process 200 provides for the control circuit 101 to access an optimized radiation treatment plan that was developed using a corresponding utility function that corresponds to a plurality of metrics. A utility function, within the context of an optimization algorithm, is a mathematical representation designed to quantify the performance, satisfaction, or preference of an agent with respect to various outcomes or states. For example, a utility function may quantify the extent to which a radiation treatment plan satisfies the required performance or preferences of a radiotherapy treatment planner. By one approach, the utility function serves as a criterion for decision-making by assigning a scalar value to each potential solution such as each potential treatment plan solution, thereby enabling the algorithm to assess and compare the relative desirability or utility of different solutions such as the relative desirability or utility of different treatment plan solutions. By systematically maximizing (or minimizing) this utility function, the optimization algorithm seeks to identify the most favorable solution (e.g., treatment plan solution) from a set of alternatives, in accordance with the specific objectives and constraints of the problem domain (such as the problem domain in a multi-criteria optimization process).

By one approach, and for the sake of a non-limiting illustrative example, the aforementioned plurality of metrics can include any combination of one or more of: (i) a metric that corresponds to a prescribed clinical goal(s) (such as a given radiation dosing goal for a target volume and/or one or more untargeted volumes such as organs-at-risk), (ii) a metric that quantifies or relates to some aspect of treatment complexity (such as dimensions of multi-leaf collimator aperture shapes or sizes relative to the target structure projection size), and/or (iii) a metric that corresponds to a preference of a treatment facility that will administer the therapeutic radiation to the particular patient. That preference may pertain, for example, to a total treatment time goal, a maximum time to devote to the planning process, or any other preference metric of choice. Other examples of preferences include, but are not limited to, patient preferences (for example, a given patient might prefer that the treatment avoid injuring a particular organ as when a musician indicates a preference that their hearing be spared as compared to their vision), particular patient-related constraint-based preferences (for example, when a given patient is unable to remain prone for more than a short period of time and this circumstance gives rise to a preference for shorter treatment times), and/or newly-available evidence of what constitutes a beneficial treatment but which might be presently considered as a deviation from current practice. This "preference of a treatment facility" can comprise an institutional preference that applies to all clinicians working within the facility and/or, if desired, can represent the personal preference(s) of a single given clinician within such a treatment facility. Any combination of one or more of these preferences may be used. The metric may comprise the extent to which the preferences are met.

At block 202, the control circuit 101 generates an approximation of the aforementioned corresponding utility function. For the sake of an illustrative example, and without intending to suggest any limitations in these regards, the description of this process 200 will presume that the approximation comprises a linear approximation. By one approach, and as exemplified in more detail below, this generation of the linear approximation of the corresponding utility function can comprise determining a tangent line at a particular point on a contour that corresponds to the aforementioned optimized radiation treatment plan that was developed using the corresponding utility function that corresponds to the plurality of metrics. In this illustrative example, the contour comprises an iso-value surface of the utility function. (As regards the nature of the "approximation," metrics referred to in a given treatment protocol are sometimes replaced in prior art optimization processes with a different metric that nevertheless correlates strongly with the original metric. The purpose of the exchange is typically to achieve better numerical performance (for example, by achieving an approach that is faster to evaluate, that has a better behaving gradient, and so forth). Those skilled in the art know to choose the new metric so that the new metric, although necessarily an approximation, will be operationally close to the original metric (or that will only require, for example, some trivial further modification such as trivial scaling that can be taken into account in the utility function).)

At optional block 203, the control circuit 101 can present, via the aforementioned user interface 103, an opportunity to select a perturbation constraint. This opportunity may comprise, for example, a field where a numerical value can be entered by the user, or, as another example, a menu or submenu of a plurality of available and user-selectable perturbation constraint values. In such a case, and as illustrated at optional block 204, the control circuit 101 can receive input, via the user interface 103, comprising a selection of the aforementioned perturbation constraint to provide a user-selected perturbation constraint.

In any event, whether the perturbation constraint is user entered or otherwise comprises a default value, at optional block 205 the control circuit 101 can access a perturbation constraint.

At block 206, the control circuit 101 then repeatedly selectively perturbs the aforementioned linear approximation, each time with respect to only a different subset of the plurality of metrics, to generate a corresponding plurality of perturbed utility functions. By one approach, this selective perturbation can be undertaken as a function of the aforementioned accessed perturbation constraint.

By one approach, the aforementioned different subset of the plurality of metrics can comprise only a single different one of the plurality of metrics. By another approach, all of the plurality of metrics are individually used to create the corresponding plurality of perturbed utility functions. Optionally, the aforementioned different subsets of the plurality of metrics can comprise any number of different ones of the plurality of metrics. If desired, the number of metrics, and which metrics to so employ, can be automatically selected, either dynamically or by utilizing a default selection in these regards. By another approach, the user can be allowed the opportunity to make one or more selections in these regards. As one illustrative example in these regards, one particular metric may comprise an always-used default selection while one or more other metrics may be selected by the user for use in combination with the default selection.

At block 207, the control circuit 101 generates a plurality of supplemental optimized radiation treatment plans, each as a function of a corresponding different one of the plurality of perturbed utility functions. (A "supplemental" optimized radiation treatment plan will be understood to be in addition to the original optimized radiation treatment plan that is initially accessed by the control circuit 101 in block 201.) As an illustrative example, if there are five different perturbed utility functions generated per the activities described at block 206, the control circuit 101 can generate five different supplemental optimized radiation treatment plans at this stage of the process 200.

It will be appreciated that these teachings can provide for generating these supplemental optimized radiation treatment plans without relying on a quadratic cost function. This approach and capability varies considerably from a typical prior art approach in these regards, which tend to rely on quadratic cost functions. (A quadratic cost function is a mathematical expression designed to evaluate the cost associated with a particular set of parameters within an algorithm, where the cost is modeled as a quadratic function of the decision variables. Specifically, this function usually takes the form of a second-degree polynomial, typically represented as f(x) *=x^{T}Qx + b^{T}x + c,* where x is the vector of decision variables, Q is a symmetric positive semidefinite matrix that defines the quadratic terms, b is a vector that defines the linear terms, and c is a scalar representing a constant term. The quadratic cost function is convex, allowing for the minimization of the function over a feasible region defined by the constraints of the optimization problem.) While certainly useful in terms of providing relevant output, the use of quadratic cost functions tends to be computationally intensive and hence requires more processing time than may be desirable or even practical in a clinical treatment setting.

It may also be observed that the present teachings also differ from previous solutions in that these described approaches can be applied to an arbitrary utility (or cost) function with no expectations regarding a certain functional form.

At block 208, the control circuit 101 can then employ the aforementioned plurality of supplemental optimized radiation treatment plans, along with the initial optimized radiation treatment plan, as a multi-criteria optimization workspace for a user to explore when selecting a particular optimized radiation treatment plan to employ when administering therapeutic radiation to a particular patient.

At optional block 209, these teachings will accommodate then administering therapeutic radiation to the particular patient (using, for example, the above-described radiation treatment platform 114 as a function of a selected one of the optimized radiation treatment plans from the multi-criteria optimization workspace).

Further details that comport with these teachings will now be presented. It will be understood that the specific details of these examples are intended to serve an illustrative purpose and are not intended to suggest any particular limitations with respect to these teachings.

A not untypical prior art approach for optimizing a radiation treatment plan provides for using a high-level utility function that can score different combinations of the different applicable aspects of interest. Unfortunately, current approaches do not readily accommodate moving from such a context to the creation of additional plans for use in a multi-criteria context. Creating a set of radiation treatment plans for multi-criteria optimization often requires applying a cost function that is a weighted sum of quadratic metrics (related but not exact to the criteria that are evaluated in the multi-criteria optimization operating space) based on making variations to the objective locations and/or weights. And unfortunately, the shape of the aforementioned high-level utility function does not readily permit direct use of the aforementioned approach to developing multi-criteria optimization plans.

The present teachings can provide for calculating a set of multi-criteria optimization plans that span a part, e.g. the relevant part, of the Pareto surface. This may be for the case when the utility function U is a function, e.g. an arbitrary (albeit a well-behaving and continuous) function, of the vector of metrics M, which are the same metrics that are going to be used in the multi-criteria optimization workflow. (Note that the utility function eventually will still depend on the plan control points and the dose calculated from them since the metrics are supposed to be, in turn, functions of the control points and dose.)

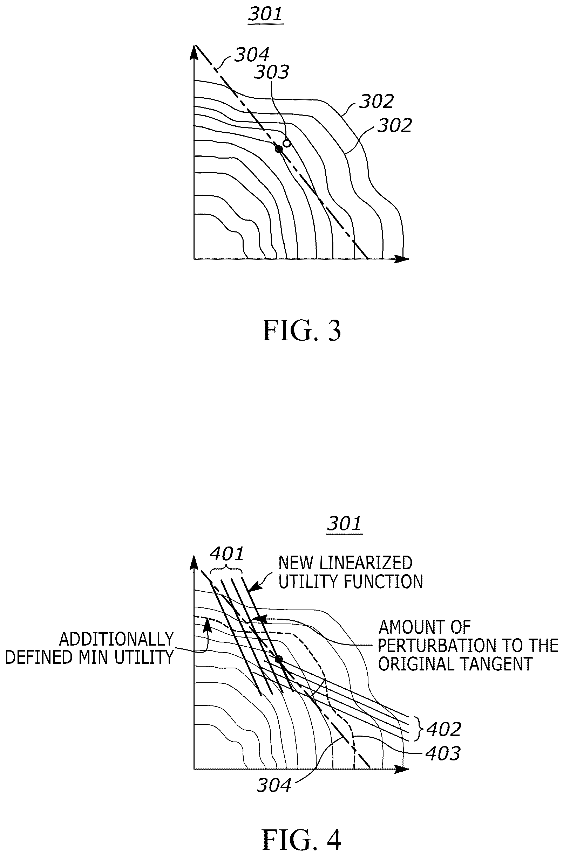

The following example illustrates the approximation of the arbitrary utility function with linear approximation. FIG. 3 presents a diagram depicting a two-dimensional metric-space 301. Any generated treatment plan can be presented as a point in this space 301 based on the values of the metrics used to calculate the plan. The corresponding utility function is presented as contours (two of which are denoted by reference numeral 302). The metrics are scaled so that an ideal plan would be in the origin (presenting, for example, a case where a target structure receives exactly the prescribed dose, while all non-targeted volumes receive no dose at all). Accordingly, a smaller metric value is better than a larger value in this context.

In this example, the optimizer has determined that a particular plan "O" 303 maximizes the utility function. The tangent line of the utility function in that point is presented as a dashed line 304.

Referring now to FIG. 4. In order to define the (yet unknown) Pareto-front, the aforementioned tangent line 304 is perturbed with a given angle for each direction (in an N-dimensional plane, the tangent-line is a (N-1) dimensional hyper-plane that can be perturbed to N directions). Additionally, if desired, one can specify a minimum utility function value.

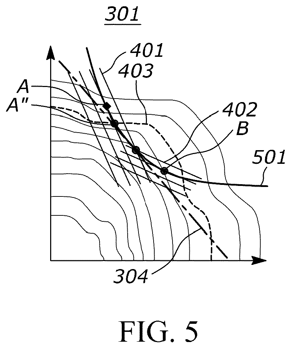

And now, referring to FIG. 5, these teachings can provide for constructing new linear perturbed utility functions and optimize supplemental plans based on those new functions (keeping the set minimum value of the original utility function as a constraint). One perturbation 401 leads to a plan A (or with the constraint A') while another perturbation 402 leads to a plan B (in this case, the constraint does not affect the solution). With these new solutions, the Pareto surface (shown as a curve denoted by reference numeral 501) is now known, and thus at least approximately facilitating the multi-criteria optimization workflow.

By one approach, the starting point of the Pareto surface generation is assumed to be that the planner has created one plan using a utility function *U(M)* defined either manually or derived automatically from clinical goals (or other similar information). The optimization process then creates the optimal plan Ξₒ = *argmax*_{Ξ} *U(M*(Ξ)), where Ξ is an arbitrary set of control points and Ξ*ₒ* is the control point set associated to the ideal plan. (Control points refer to specific positions or angles at which a radiation beam is turned on or off during treatment. Control points typically serve to help shape the radiation beam and to optimize the dose distribution to target the tumor while sparing surrounding healthy tissues.)

To continue with this example, the planner now wants to start the multi-criteria optimization workflow to evaluate the possible trade-offs around the optimized plan. In particular, the planner now seeks an approximation for the Pareto surface in the vicinity of the created plan. By one approach, a first stage is to define a linear approximation of the utility function as ${U}_{o}^{L} \left(M\right) = {β}_{o} M$*,* where *βₒ = ∇_{M}U(M*(Ξ*ₒ*)) is the gradient direction of the original utility function at the location(Ξ*ₒ*) (that is, the found minimum solution). The dashed line 304 in FIG. 3 presents the tangent line that is perpendicular to that gradient direction.

Provided that the user wishes to explore the multi-criteria optimization workflow using all the metrics in M, these teachings can provide for constructing a new utility function by perturbing one dimension at a time (at least in some application settings, this approach can also be effective if only a subset of metrics is selected for the multi-criteria optimization workflow by omitting perturbations that are not useful/interesting; it would also be possible to use an increased number of plans to construct a larger - or more accurate - region of the Pareto surface).

The perturbed utility functions can have the form ${U}_{i}^{L} \left(M\right) = {β}_{i} M$ where i = 1 ... N (where N is the number of metrics in M) (accordingly, the number of perturbations can be the same as the dimensionality of the metric-space), and *βᵢ* = *βₒ* + *δ*ᵢ. The most simple choice for the perturbation *δᵢ* is perhaps to perturbate only one metric value at a time (for example, in component form ${β}_{i} = \left\{{β}_{o}^{1},…,{β}_{o}^{i}+{δ}^{i},…,{β}_{o}^{N}\right\}$ ) (see, for example, the contours denoted by reference numerals 401 and 402 in FIG. 4). The scalar perturbation factor δⁱ is essentially an arbitrary parameter representing how much to emphasize metric *Mᵢ* in this perturbation. To select reasonable strength of the perturbation, these teachings will optionally accommodate utilizing heuristic rules or user given information (for example, the difference between "per protocol" and "acceptable variation" associated to a goal related to the metric). Note that here it is presumed that the perturbations were created for one metric at a time, but these teachings will accommodate multiple ways to do N linearly independent perturbations.

Additionally, the user can constrain how far the Pareto surface search reaches by setting a minimum value for the utility function *Û < U(M*(Ξ)) as a constraint (as exemplified, for example, by the contour line in FIGS. 4 and 5 that is denoted by reference numeral 403).

For each perturbed utility function, a new plan can be created by optimizing ${Ξ}_{i} = {argmax}_{Ξ} {U}_{i}^{L} \left(M\left(Ξ\right)\right)$ (with the possible constraint *U(M*(Ξ)) *> Û).*

Note that, similar to current multi-criteria optimizations solutions, a more accurate Pareto-surface modeling might be desirable, and this could lead to schemas where more than one plan is created for each dimension in the multi-criteria optimization navigation space. By one approach, this need can be achieved by using multiple perturbation strengths *δⁱ* (or their combinations) to obtain more variations of the linearized utility function.

The foregoing approaches utilize a simplified utility function based on the linear approximation of the original function. These teachings will also support, however, employing a non-linear perturbation approach. For example, the perturbation to the original utility functions can be expressed as ${U}_{i}^{P} \left(M\right) = U \left(M\right) - {U}_{o}^{L} \left(M\right) + {U}_{i}^{L} \left(M\right)$*.* In the previously presented example, the linearization scheme provides for ${U}_{i}^{P} \left(M\left(Ξ\right)\right) =$ *U(M()) + 6ᵢM,* where *δᵢ* = 0, ...,δⁱ, ...,0}. This function adds a linear component to a single dimension of the metric while keeping the original utility function otherwise unmodified. The construction of the Pareto surface is done similarly than in the previous case except that ${U}_{i}^{P}$ is used instead of ${U}_{i}^{L}$.

Note that there are also other possibilities to define the perturbation, for example, in a way that restricts the perturbation to the neighborhood of the solution Ξ*ₒ*. As one example, these teachings will accommodate using an error function. With this, the perturbed utility function becomes $U \left(M\left(Ξ\right)\right) + {δ}^{i} erf \left(\frac{{M}_{o}^{i} - {M}^{i}}{c}\right)$. Here, constant c determines the width of the neighborhood for the modification.

Further aspects of these teachings are provided by the subject matter of the following clauses (where it will be understood that any of these clauses can be combined with any of one or more of the other clauses as desired).

Clause 1. A method comprising: by a control circuit: accessing an optimized radiation treatment plan that was developed using a corresponding utility function that corresponds to a plurality of metrics; generating an approximation of the corresponding utility function; repeatedly selectively perturbing the approximation, each time with respect to only a different subset of the plurality of metrics, to generate a corresponding plurality of perturbed utility functions; generating (e.g. automatically generating) a plurality of supplemental optimized radiation treatment plans, each as a function of a corresponding different one of the plurality of perturbed utility functions; employing the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient.

Clause 2. The method of clause 1 wherein the plurality of metrics include at least one of a metric that corresponds to a prescribed clinical goal and a metric that corresponds to a preference of a treatment facility that will administer the therapeutic radiation to the particular patient.

Clause 3. The method of clause 1 or clause 2 wherein generating the plurality of supplemental optimized radiation treatment plans comprises generating the plurality of supplemental optimized radiation treatment plans without relying on a quadratic cost function.

Clause 4. The method of any preceding clause wherein the different subset of the plurality of metrics comprises only a single different one of the plurality of metrics.

Clause 5. The method of clause 4 wherein all of the plurality of metrics are individually used to create the corresponding plurality of perturbed utility functions.

Clause 6. The method of any preceding clause wherein generating the approximation of the corresponding utility function comprises determining a tangent line at a particular point on a contour that corresponds to the optimized radiation treatment plan that was developed using the corresponding utility function that corresponds to the plurality of metrics.

Clause 7. The method of clause 6 wherein perturbing the approximation comprises angularly perturbing the tangent line with respect to the particular point.

Clause 8. The method of clause 7 further comprising: accessing a perturbation constraint; and wherein perturbing the approximation by angularly perturbing the tangent line with respect to the particular point includes limiting angular perturbation as a function of the perturbation constraint.

Clause 9. The method of clause 8 further comprising: presenting, via a user interface, an opportunity to select the perturbation constraint; receiving input, via the user interface, comprising a selection of the perturbation constraint to provide a user-selected perturbation constraint; and wherein accessing a perturbation constraint comprises accessing the user-selected perturbation constraint.

Optional Clause 10. The method of any preceding clause further comprising: administering therapeutic radiation to the particular patient as a function of a selected one of the optimized radiation treatment plans from the multi-criteria optimization workspace.

Clause 11. An apparatus comprising: a control circuit configured to: access an optimized radiation treatment plan that was developed using a corresponding utility function that corresponds to a plurality of metrics; generate an approximation of the corresponding utility function; repeatedly selectively perturb the approximation, each time with respect to only a different subset of the plurality of metrics, to generate a corresponding plurality of perturbed utility functions; generate (e.g. automatically generate) a plurality of supplemental optimized radiation treatment plans, each as a function of a corresponding different one of the plurality of perturbed utility functions; employ the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient.

Clause 12. The apparatus of clause 11 wherein the plurality of metrics include at least one of a metric that corresponds to a prescribed clinical goal and a metric that corresponds to a preference of a treatment facility that will administer the therapeutic radiation to the particular patient.

Clause 13. The apparatus of clause 11 or clause 12 wherein the control circuit is configured to generate the plurality of supplemental optimized radiation treatment plans by generating the plurality of supplemental optimized radiation treatment plans without relying on a quadratic cost function.

Clause 14. The apparatus of any of clause 11 to clause 13 wherein the different subset of the plurality of metrics comprises only a single different one of the plurality of metrics.

Clause 15. The apparatus of clause 14 wherein all of the plurality of metrics are individually used to create the corresponding plurality of perturbed utility functions.

Clause 16. The apparatus of any of clause 11 to clause 15 wherein the control circuit is configured to generate the approximation of the corresponding utility function by determining a tangent line at a particular point on a contour that corresponds to the optimized radiation treatment plan that was developed using the corresponding utility function that corresponds to the plurality of metrics.

Clause 17. The apparatus of clause 16 wherein the control circuit is configured to perturb the approximation by angularly perturbing the tangent line with respect to the particular point.

Clause 18. The apparatus of clause 17 wherein the control circuit is further configured to: access a perturbation constraint; and wherein the control circuit is configured to perturb the approximation by angularly perturbing the tangent line with respect to the particular point and by limiting angular perturbation as a function of the perturbation constraint.

Clause 19. The apparatus of clause 18 wherein the control circuit is further configured to: present, via a user interface, an opportunity to select the perturbation constraint; receive input, via the user interface, comprising a selection of the perturbation constraint to provide a user-selected perturbation constraint; and wherein the control circuit is configured to access the perturbation constraint by accessing the user-selected perturbation constraint.

Clause 20. The apparatus of any of clause 11 to clause 19 wherein the control circuit is further configured to: administer therapeutic radiation to the particular patient as a function of a selected one of the optimized radiation treatment plans from the multi-criteria optimization workspace.

Clause 21. A non-transitory computer-readable medium comprising instructions stored thereon, which instructions, when executed on a processor, perform the steps of: accessing an optimized radiation treatment plan that was developed using a corresponding utility function that corresponds to a plurality of metrics; generating an approximation of the corresponding utility function; repeatedly selectively perturbing the approximation, each time with respect to only a different subset of the plurality of metrics, to generate a corresponding plurality of perturbed utility functions; generating a plurality of supplemental optimized radiation treatment plans, each as a function of a corresponding different one of the plurality of perturbed utility functions; employing the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient;.
and optionally:
wherein the instructions, when executed on the processor, perform the method of any of clause 2 to clause 10.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method comprising:
by a control circuit:
accessing an optimized radiation treatment plan that was developed using a corresponding utility function that corresponds to a plurality of metrics;
generating an approximation of the corresponding utility function;
repeatedly selectively perturbing the approximation, each time with respect to only a different subset of the plurality of metrics, to generate a corresponding plurality of perturbed utility functions;
generating a plurality of supplemental optimized radiation treatment plans, each as a function of a corresponding different one of the plurality of perturbed utility functions;
employing the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient.

2. The method of claim 1 wherein the plurality of metrics include at least one of a metric that corresponds to a prescribed clinical goal and a metric that corresponds to a preference of a treatment facility that will administer the therapeutic radiation to the particular patient.

3. The method of claim 1 or claim 2 wherein generating the plurality of supplemental optimized radiation treatment plans comprises generating the plurality of supplemental optimized radiation treatment plans without relying on a quadratic cost function.

4. The method of any preceding claim wherein the different subset of the plurality of metrics comprises only a single different one of the plurality of metrics;
and optionally:
wherein all of the plurality of metrics are individually used to create the corresponding plurality of perturbed utility functions.

5. The method of any preceding claim wherein generating the approximation of the corresponding utility function comprises determining a tangent line at a particular point on a contour that corresponds to the optimized radiation treatment plan that was developed using the corresponding utility function that corresponds to the plurality of metrics.

6. The method of claim 5 wherein perturbing the approximation comprises angularly perturbing the tangent line with respect to the particular point.

7. The method of claim 6 further comprising:
accessing a perturbation constraint;
and wherein perturbing the approximation by angularly perturbing the tangent line with respect to the particular point includes limiting angular perturbation as a function of the perturbation constraint;
and optionally:
the method further comprising:
presenting, via a user interface, an opportunity to select the perturbation constraint;
receiving input, via the user interface, comprising a selection of the perturbation constraint to provide a user-selected perturbation constraint; and
wherein accessing a perturbation constraint comprises accessing the user-selected perturbation constraint.

8. An apparatus comprising:
a control circuit configured to:
access an optimized radiation treatment plan that was developed using a corresponding utility function that corresponds to a plurality of metrics;
generate an approximation of the corresponding utility function;
repeatedly selectively perturb the approximation, each time with respect to only a different subset of the plurality of metrics, to generate a corresponding plurality of perturbed utility functions;
generate a plurality of supplemental optimized radiation treatment plans, each as a function of a corresponding different one of the plurality of perturbed utility functions;
employ the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient.

9. The apparatus of claim 8 wherein the plurality of metrics include at least one of a metric that corresponds to a prescribed clinical goal and a metric that corresponds to a preference of a treatment facility that will administer the therapeutic radiation to the particular patient.

10. The apparatus of claim 8 or claim 9 wherein the control circuit is configured to generate the plurality of supplemental optimized radiation treatment plans by generating the plurality of supplemental optimized radiation treatment plans without relying on a quadratic cost function.

11. The apparatus of any of claim 8 to claim 10 wherein the different subset of the plurality of metrics comprises only a single different one of the plurality of metrics;
and optionally:
wherein all of the plurality of metrics are individually used to create the corresponding plurality of perturbed utility functions.

12. The apparatus of any of claim 8 to claim 11 wherein the control circuit is configured to generate the approximation of the corresponding utility function by determining a tangent line at a particular point on a contour that corresponds to the optimized radiation treatment plan that was developed using the corresponding utility function that corresponds to the plurality of metrics;
and optionally:
wherein the control circuit is configured to perturb the approximation by angularly perturbing the tangent line with respect to the particular point.

13. The apparatus of claim 12 wherein the control circuit is further configured to:
access a perturbation constraint;
and wherein the control circuit is configured to perturb the approximation by angularly perturbing the tangent line with respect to the particular point and by limiting angular perturbation as a function of the perturbation constraint;
and optionally:
wherein the control circuit is further configured to:
present, via a user interface, an opportunity to select the perturbation constraint;
receive input, via the user interface, comprising a selection of the perturbation constraint to provide a user-selected perturbation constraint; and
wherein the control circuit is configured to access the perturbation constraint by accessing the user-selected perturbation constraint.

14. The apparatus of any of claim 8 to claim 13 wherein the control circuit is further configured to:
administer therapeutic radiation to the particular patient as a function of a selected one of the optimized radiation treatment plans from the multi-criteria optimization workspace.

15. A non-transitory computer-readable medium comprising instructions stored thereon, which instructions, when executed on a processor, perform the steps of:
accessing an optimized radiation treatment plan that was developed using a corresponding utility function that corresponds to a plurality of metrics;
generating an approximation of the corresponding utility function;
repeatedly selectively perturbing the approximation, each time with respect to only a different subset of the plurality of metrics, to generate a corresponding plurality of perturbed utility functions;
generating a plurality of supplemental optimized radiation treatment plans, each as a function of a corresponding different one of the plurality of perturbed utility functions;
employing the plurality of supplemental optimized radiation treatment plans along with the optimized radiation treatment plan as a multi-criteria optimization workspace for a user to explore when selecting a particular optimized radiation treatment plan to employ when administering therapeutic radiation to a particular patient;
and optionally:
wherein the instructions, when executed on the processor, perform the method of any of claim 2 to claim 7.
